# EUROPEAN PATENT SPECIFICATION

(11) **EP 3 498 339 B1**
(45) Date of publication and mention of the grant of the patent: **24.09.2025**
(21) Application number: 19155503.6
(22) Date of filing: 12.08.2013
(51) Int. Cl.: A62B 18/00, A62B 18/10, A61M 16/06

(54) **POWERED EXHAUST APPARATUS FOR A PERSONAL PROTECTION RESPIRATORY DEVICE**
ELEKTRISCHE ABLUFTVORRICHTUNG FÜR EIN PERSONENATEMSCHUTZGERÄT
APPAREIL D'ÉCHAPPEMENT ÉLECTRIQUE POUR UN DISPOSITIF RESPIRATOIRE DE PROTECTION PERSONNELLE

(30) Priority: 31.08.2012 GB 201215568
(43) Date of publication of application: 19.06.2019
(62) Divisional of application: 13752764.4
(73) Proprietor: 3M Innovative Properties Company, Saint Paul, MN 55133-3427 (US)
(72) Inventor: CURRAN, Desmond T., Durham, County Durham DH1 4NU (GB); HENDERSON, Christopher P., Bracknell, Berkshire RG12 8HT (GB); COOPER, Benjamin H., Bracknell, Berkshire RG12 8HT (GB); GODFREY, Phillip, J., Teesville, Middlesbrough NS6 0AN (GB)
(74) Representative: Mathys & Squire

(56) References cited:
- WO-A1-90/05565
- CN-A- 1 189 384
- JP-A- 2008 295 993
- KR-U- 20100 003 793
- US-A- 2 891 541
- US-A1- 2006 076 012
- US-B1- 6 257 235

## Description

### TECHNICAL FIELD OF THE INVENTION

The present invention relates to an exhaust apparatus for personal protection respiratory devices, particularly negative pressure respirators. In particular, the present invention relates to a powered exhaust apparatus which can be releasably connected to a personal protection respiratory device. In use, the powered exhaust apparatus removes the hot and moist air that can often build-up inside a negative pressure respirator to significantly improve and enhance wearer comfort.

### BACKGROUND

Negative pressure respirators are well known in the art. With respirators of this type, filtered air is drawn into the enclosed space between the inside of the respirator and a wearer's face through a filter system by the wearer's breathing action. When the wearer draws a breath, negative pressure is created in the respirator and air is drawn in through the filter system. When the wearer exhales a breath, spent air leaves the respirator through an exhalation valve and/or back through the filter system.

Although negative pressure respirators are available in many different configurations, and offer many different benefits, they all have one major drawback, that of the uncomfortable build-up of heat and moisture that can sometimes occur inside the respirator. The heat and moisture build-up is caused by the trapping of the wearer's exhaled breath in the cavity created between the respirator and the wearer's face. As the wearer works harder, and/or wears the respirator for extended periods of time, heat and moisture build-up may increase.

Many different solutions have been proposed in the prior art to eliminate, or at least minimise, the problem of heat and moisture build-up inside negative pressure respirators. For example, the addition of exhalation valves, and optimising the operation of these exhalation valves. The design and optimisation of low pressure drop filters and media has also been proposed to alleviate this problem and/or by controlling the filter surface area and filter material pressure drop. Another solution in the prior art is to include pads to absorb the moisture.

US 2 891541 A describes a respiratory safety face mask comprising a flexible body adapted to cover the face, eye socket openings in said mask covered by eye glass lenses, concavo-convex inwardly extending ribs surrounding each opening and having a body attached leg wall and a free edge wall joined by a bight adapted to be seated around each eye, cheekside and nose bridge side openings, respectively, in said body attached leg wall, inhalation intake air cheek channel means for conducting air to said cheekside openings, and nose channel means communicating with said nose bridge side openings for causing intake air to flow across said eye openings.

WO 90/05565 A1 describes a protective hood for use either in fire emergencies or as protection against escaping irritant gases in an industrial environment, comprising a bag-like head covering of heat resistant, and/or gas impermeable sheet material, the bag being transparent over at least the area which, in use, falls in front of the eyes of the wearer and having a filter pad or pads positioned to cover the nose and mouth of the wearer and attached in such manner that the wearer can inhale therethrough, sealing means being provided at the mouth of the bag to create a seal around the neck of the wearer when donned. The hood is fitted with at least one motor-driven fan to assist in removal of accumulating exhaled gases within the hood and with power means to drive the motor, the fan and the power means being arranged in the hood in such manner that power is supplied to the motor automatically when the hood is unpacked or first donned.

US 6 257 235 B1 describes a face mask, comprising: a filter body having a shape so as to cover at least the nose and mouth of a wearer, said filter body defined by peripheral edges, said filter body being breathable so that the wearer inhales and exhales through said filter body; said peripheral edges configured for substantially sealing engagement against the wearer's skin; said filter body further comprising an exterior surface, and an interior surface defining an interior air volume adjacent the wearer's nose and mouth; a securing mechanism attached to said filter body and configured to hold said filter body on the wearer's skin; and a fan configured directly against said exterior surface of said filter body so that when operated said fan draws at least a portion of its air flow directly through said filter body thereby communicating with said interior air volume through said filter body, wherein said fan is operational in a direction to draw air exhaled by the wearer from within said interior air volume.

Despite many years of development work, wearers of negative pressure respirators may still experience problems with heat and moisture build-up.

Accordingly it is therefore desirable to be able to find a way to ensure that negative pressure respirators can be worn comfortably for an extended period of time, regardless of the ambient temperature or weather conditions, and the type and intensity of the work being undertaken.

### SUMMARY OF THE INVENTION

The present invention aims to address these issues by providing an exhaust apparatus for releasable connection to a personal protection respiratory device that defines a filtered air volume adjacent to the face of a wearer and comprises at least one exhalation valve, the exhaust apparatus comprising:
a blower comprising an inlet, a motor fan assembly, and an outlet;
an attachment means for releasably connecting the blower to the at least one exhalation valve, the blower being in fluid connection with the at least one exhalation valve when releasably connected thereto and being operable to draw a portion of the wearer's exhaled breath through the at least one exhalation valve; and
a secondary exhalation valve positioned between the inlet of the blower and the motor fan assembly.

An advantage of using an exhaust apparatus for releasable connection to a personal protection respiratory device is that it improves the comfort and overall experience for the wearer regardless of the intensity of the work being undertaken. The benefit is noticeable as soon as the blower is operated, even if the wearer is undertaking a low intensity task. Use of the present invention especially allows the respirator to be worn for intensive work, and/or for long periods of time, and/or in hot and humid environmental conditions by removing the heat and moisture build-up inside the respirator.

Advantageously, the use of a powered exhaust apparatus which draws the hot air and moisture out of the enclosed space between the inside of the respirator and the wearer, means that the difficulties sometimes experienced in hot and humid conditions or after extended periods of use are minimized or removed completely. The act of drawing the hot and moist air out of the respirator and replacing it with fresh un-breathed filtered air also makes breathing easier for the wearer. This is because the first portion of the next breath of the wearer is fresh un-breathed filtered air, rather than the last portion of the previously exhaled breath. Since the present invention draws more air out of the respirator than wearer exhales, the difference is fresh air drawn in through filters. This also gives improvements in terms of the carbon dioxide levels inside the respirator.

Further the attachment means may be selected from a group consisting of interference fit, screw thread, snap fit engagement, bayonet, quick release mechanism, slider and groove engagement, locking pin, locking clip and mechanical hook and loop fastener.

Preferably the personal protection respiratory device is selected from a group consisting of disposable, reusable, half mask, full face, particulate, gas and vapour and tight-fitting hood respirators.

The blower may further be operable at a volumetric flow rate of between 0 to 180 litres per minute.

The blower of the exhaust apparatus may be operable to favorably allow for a reduction of the pressure inside the personal protection respiratory device by at least 150 Pa at the peak exhalation flow rate of the wearer, or a reduction of the temperature inside the personal protection respiratory device by at least about 1°C to 3°C, or a reduction of the rebreathed carbon dioxide level inside the personal protection respiratory device by up to about 0.7%.

The exhaust apparatus may further comprise a portable power supply for the blower, the portable power supply being either integrally mounted with the blower or remotely positionable on the wearer.

Preferably the blower is in fluidic connection with at least one exhalation valve via a breathing hose, tube, pipe, duct or channel.

Further the secondary exhalation valve may be integrally formed with the exhaust apparatus.

Preferably the secondary exhalation valve comprises a valve seat that includes a seal surface and a flexible flap.

The exhaust apparatus of the present invention may be suitable for use with a filtering respirator having an exhalation valve, wherein the exhaust apparatus draws filtered air out of the enclosed space between the inside of the filtering respirator and a wearer though the exhalation valve.

The exhaust apparatus of the present invention may be suitable for use with a respirator, comprising a mask body that comprises a filtering system, the mask body being dimensioned to define a filtered air volume adjacent to the face of a wearer, the mask body further comprises at least one exhalation valve for allowing exhalation of the wearer's exhaled breath. The respirator may further comprise an air distribution manifold in fluid connection with the filtering system.

### BRIEF DESCRIPTION OF THE DRAWINGS

The present invention will now be described by way of example only, and with reference to the accompanying drawings, in which:
Figure 1 is an exploded view of a personal protection respiratory device 20 and an exhaust apparatus 10 not according to the claimed invention for releasable connection to the personal protection respiratory device 20;
Figure 2 shows a front side perspective view of the exhaust apparatus 10 of Figure 1 connected to the personal protection respiratory device 20;
Figure 3 is a cross-sectional side view of the exhaust apparatus 10 of Figure 1 taken along the dashed line A'-A" in Figure 6;
Figure 4 schematically illustrates a sectional side view of the exhaust apparatus 10 of Figure 1 connected to the personal protection respiratory device 20 indicating the operation to draw a portion of the wearer's 100 exhaled breath through a exhaust valve 26 on the personal protection respiratory device 20;
Figure 5 is a side view of the exhaust apparatus 10 of Figure 1 connected to the personal protection respiratory device 20;
Figure 6 shows a front view of the exhaust apparatus 10 of Figure 1 connected to the personal protection respiratory device 20;
Figure 7 is a rear side perspective view of the exhaust apparatus 10 of Figure 1;
Figure 8 illustrates a front side perspective view of an exemplary exhaust apparatus 10 further showing a remotely positionable battery pack 46;
Figure 9 is a sectional side view of an exemplary exhaust apparatus 10 according to the present invention including a secondary exhalation valve 58 which reduces the exhalation pressure drop when the exhaust apparatus 10 is not powered;
Figure 10 shows a front view of the exhaust apparatus 10 of Figure 9 being connected to a full facepiece respiratory device 70;
Figure 11 is a sectional side view of the exhaust apparatus 10 of Figure 9 connected to a full facepiece respiratory device 70;
Figure 12 is a graph showing the average temperature recorded inside a 3M^{™} 4251 Valved Filtering Half Face Respirator as a function of the voltage being applied to an exhaust apparatus 10 connected thereto;
Figure 13 illustrates a graph of the rebreathed carbon dioxide measured inside a 3M^{™} 4251 Valved Filtering Half Face Respirator as a function of the voltage being applied to an exhaust apparatus 10 connected thereto;
Figure 14 is a graph of the measured pressure inside a standard 3M^{™} 4251 Valved Filtering Half Face Respirator using a breathing machine set at 30 litres per minute, compared to a 3M^{™} 4251 Valved Filtering Half Face Respirator having an exhaust apparatus 10 connected thereto;
Figure 15 is a graph showing the exhalation pressure drop obtained from a standard 3M^{™} 4251 Valved Filtering Half Face Respirator, along with measurements of the exhalation pressure drop of a 3M^{™} 4251 Valved Filtering Half Face Respirator with an exhaust apparatus 10 connected but no power supplied, along with an exhaust apparatus 10 fitted with a secondary exhalation valve 58;
Figure 16 illustrates the measured exhalation pressure drop using a 3M^{™} 4251 Valved Filtering Half Face Respirator having an exhaust apparatus 10 connected thereto as a function of flow rate and applied voltage; and
Figure 17 is a graph of the rebreathed carbon dioxide measured inside a 3M^{™} 6800 Full Facepiece Reusable Respirator as a function of the voltage being applied to an exhaust apparatus 10 connected thereto, with and without an inner face cup.

### DETAILED DESCRIPTION OF THE PREFERRED EMBODIMENTS

The present invention has adopted the approach of using an exhaust apparatus for releasable or permanent connection to a personal protection respiratory device such that it improves the comfort and overall experience for the wearer. Use of the present invention allows the respirator to be worn for intensive work, and/or for long periods of time, and/or in hot and humid environmental conditions by removing the heat and moisture build-up inside the respirator. The benefit felt by the wearer occurs both at very low work rates, e.g. whilst performing sedentary tasks, but the effect can also be increased as work rate increases. The use of a powered exhaust apparatus which draws the hot air and moisture out of the enclosed space between the inside of the respirator and the wearer, means that the difficulties sometimes experienced in hot and humid conditions or after extended periods of use are minimized or removed completely. Advantageously, the act of drawing the hot and moist air out of the respirator and replacing it with fresh un-breathed air also makes breathing easier for the wearer. This is because the first portion of the next breath of the wearer is fresh un-breathed air, rather than the last portion of the previously exhaled breath. This also gives improvements in terms of the carbon dioxide levels inside the respirator.

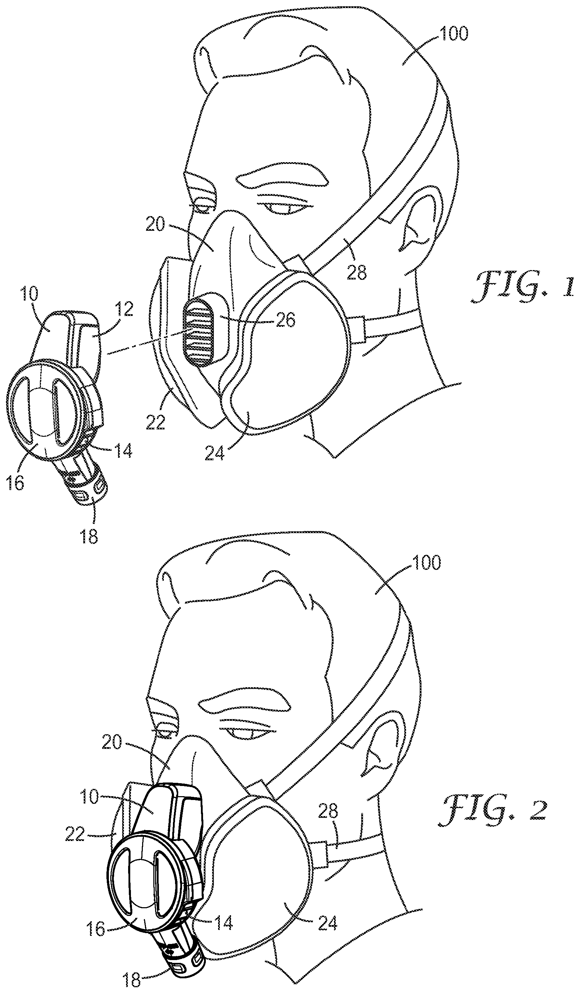

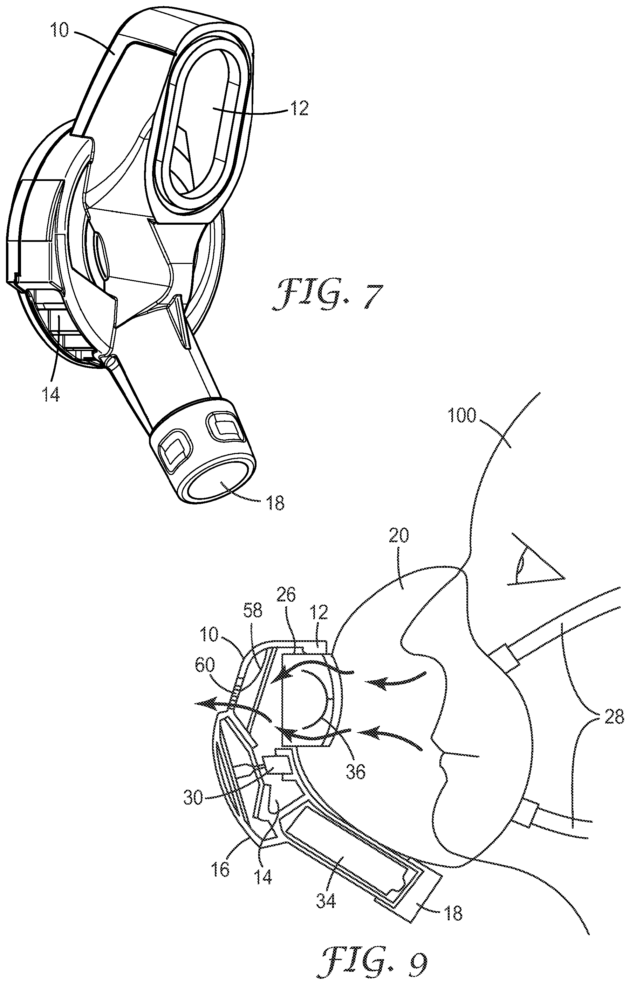

Figure 1 is an exploded view of a personal protection respiratory device 20 and an exhaust apparatus 10 not according to the claimed invention but which is helpful in the general understanding of exhaust apparatuses according to the claimed invention. The exhaust apparatus 10 of Figure 1 is able to releasably connect or engage with a personal protection respiratory device 20. Whilst the respirator 20 that is illustrated in Figures 1, 2, 4, 5, 6, and 8 as well as in Figure 9, Figure 9 illustrating an exemplary exhaust apparatus 10 according to the claimed invention, is indicative of the 3M^{™} 4000 Series of gas, vapor and particulate respirators, the exhaust apparatus 10 of the present invention can be utilized with any negative pressure respiratory device 20. The skilled person will appreciate that the term "respirator" or "respiratory mask", as used interchangeably herein, is intended to mean a breathing device worn to prevent the inhalation of hazardous substances, particles, vapors or noxious gases. The term "negative pressure respiratory mask" is intended to cover any respirator in which the air pressure inside the mask becomes lower than the ambient air pressure when the wearer inhales.

A negative pressure respiratory mask 20 as described herein is used to mean any form of respirator intended to fit the face of the wearer 100 in a substantially sealed configuration causing the air inhaled and exhaled by the wearer 100 to pass through a filter body or a filter portion of the respirator. Negative pressure respiratory mask 20 can also be a full or half facepiece mask, depending upon the hazard of concern. Again, these masks utilize a filter which prevents the inhalation of contaminants, particles, gases and vapors from the air inhaled by the wearer. Some common examples of this type of respirator are manufactured by 3M Company located in St. Paul, Minnesota, and include the 3M^{™} 6000 and 7000 Series of reusable respirators or tight-fitting hood facepiece respirators.

Disposable respirators, such as the 3M^{™} 8000 and 9000 Series of cup-shaped and flat-folded products, are lightweight single-piece respirators that employ a filter media which removes particulates and mists from the air stream as the wearer draws a breath. The entire unit is designed to be discarded after some extended period or a single use or single shift, depending on the contaminant. Filtering facepieces, such as the 3M^{™} 6000 and 7000 Series are generally reusable products and which can have replaceable filter cartridges. Typically one or two cartridges attach securely to half mask or full facepiece which has built into it a corresponding number of valves for inhalation, and usually one for exhalation.

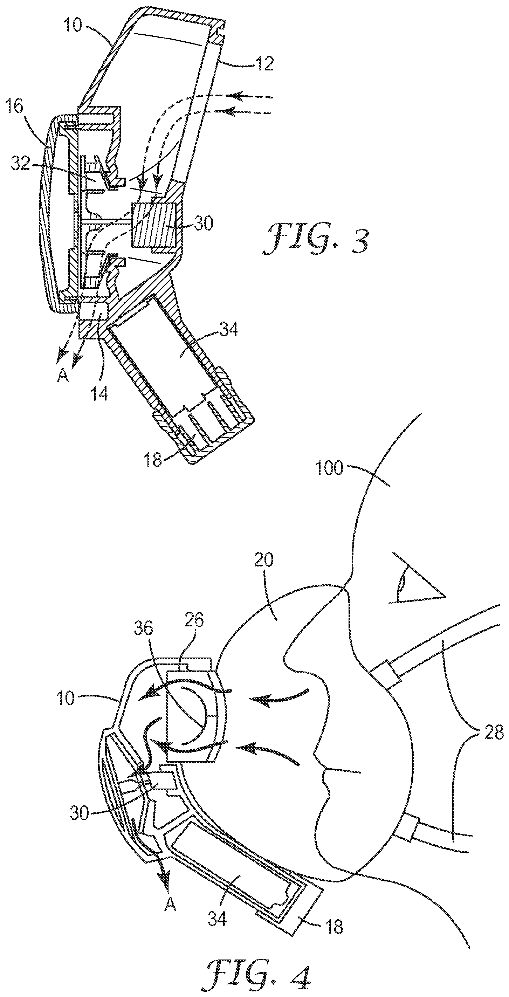

The personal protection respiratory device 20 that is illustrated in Figure 1 is a 3M^{™} 4251 Valved Filtering Half Face Respirator. As shown in Figure 1, a pair of filter cartridges 22, 24 are integrally attached to the respirator mask 20 at respective inhalation ports (not shown). Each of the inhalation ports having a respective inhalation valve (not shown) on the inside of the respirator mask 20 which open as a wearer 100 draws a breath. The face mask 20 has an exhaust valve 26 with a one-way exhalation valve diaphragm (shown as reference numeral 36 in Figure 4) and adjustable straps 28 for attachment to the wearer 100.

The respiratory mask 20 has a conformable gasket or seal which generally encloses the wearer's 100 mouth and nose. Since a good seal is needed to ensure filtration of the containments one major drawback is that sometimes an uncomfortable build-up of heat and moisture is noticed by the wearer 100 inside the respirator 20. As the wearer 100 works harder, and or wears the respirator 20 for extended periods of time, heat and moisture build-up can occur. The heat and moisture build-up is caused by the trapping of the exhaled breath in the cavity created between the respirator 20 and the wearer's 100 face.

As further illustrated in Figures 1 and 2, the illustrated exhaust apparatus 10 has a generally elongate form. The exhaust apparatus 10 includes an inlet 12 (which is shown more clearly in Figure 7) and a series of openings which define an outlet 14. Positioned between the inlet 12 and the outlet 14 is a blower which is contained inside housing 16. The blower is a motor fan assembly, as shown in more detail in Figure 3. To control the operation of the blower, a switch mechanism 18 is accessible to the wearer 100. The switch mechanism 18 can have a simple on/off mode of operation or can include a variable adjustment so that the wearer 100 can optimize the desired blower speed, and hence, cooling effect based upon the environmental conditions, the task the wearer 100 is undertaking, and the wearer's personal choice.

A cooling effect is achieved by the use of such an exhaust apparatus 10 as described further herein. When a wearer 100 inhales a breath, "cooler" ambient air is drawn into the respiratory mask 20 either though the filter cartridges 22, 24 as shown in Figures 1 and 2 for a reusable mask, or through, for example, a filter portion or filtering mask body of the respirator, as with a disposable mask. Heat and moisture build-up is then caused by trapping the exhaled breath in the cavity created between the respirator 20 and the wearer's 100 face. When operated, the exhaust apparatus 10 of the present invention draws this warm and moist air out through the exhaust valve 26 and replaces it with fresh "cooler" un-breathed filtered air, and reduces the exhalation breathing resistance, as described below. This produces a noticeable cooling benefit for the wearer 100.

The exhaust apparatus 10 solves this problem because it draws the hot air and moisture out of the enclosed space between the inside of the respirator 20 and the wearer 100. The act of drawing the hot and humid air out of the respirator 20 and replacing it with fresh un-breathed filtered air also makes breathing easier for the wearer 100. This is because the first portion of the next breath of the wearer 100 is fresh un-breathed air, rather than the last portion of the previously-exhaled breath. This also gives improvements in terms of carbon dioxide reduction inside the mask 20.

The skilled person will appreciate that since the exhaust apparatus 10 is fluidically connected to the exhaust valve 26 on the respiratory mask 20 any overbreathing of the blower (i.e., back flow through the blower caused by inhalation by the wearer 100) is prevented by the one-way exhaust valve 26 on the respiratory mask 20. Positioning the exhaust apparatus 10 on the one-way exhaust valve 26 ensures that no contaminants, particulates, mists, vapors or gases are inhaled by the wearer 100 and the integrity of the personal protection respiratory device 20 is maintained. The exhaust apparatus 10 is designed to create just enough air flow and pressure to generate the cooling effect, which enables the unit to be made small and light enough to be attached to even a disposable fabric respirator, in fact any respirator that includes an exhaust valve 26.

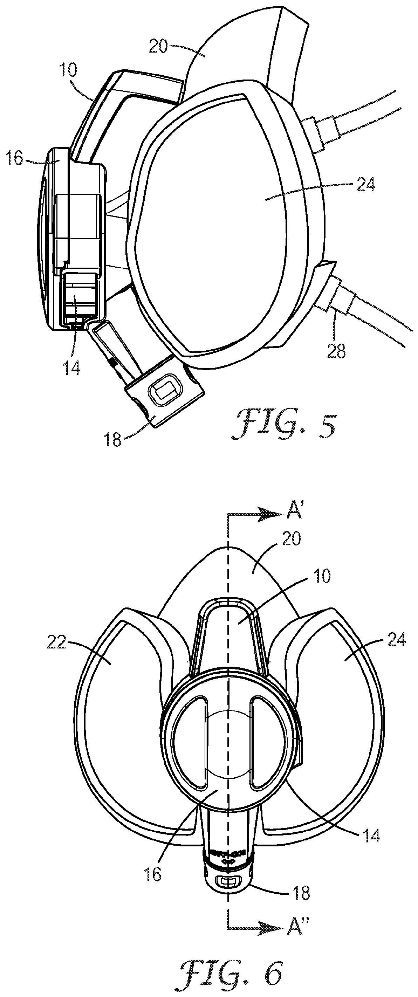

Figure 3 shows further detail on the operation of the exhaust apparatus 10 of Figure 1 and is a cross-sectional side view of the exhaust apparatus 10 taken along the dashed line A'-A" in Figure 6.

Exhaust apparatuses of the present invention include a blower, said blower including an inlet, a motor fan assembly and an outlet, as well as an attachment means for releasably connecting the blower to the at least one exhalation valve, the blower being in fluid connection with the at least one exhalation valve when releasably connected thereto and being operable to draw a portion of the wearer's exhaled breath through the at least one exhalation valve.

The inlet 12 of the exhaust apparatus 10 may be shaped to releasably connect by way of an interference fit to the shape and dimensions of the respective exhaust valve 26 situated on the respiratory mask 20. Whilst the exhaust apparatus 10 may be connected by way of an interference fit, the skilled person will appreciate that any form of releasable connection to the exhaust valve 26 is possible, including, for example, connection by way of a screw thread, snap fit engagement, bayonet, quick release mechanism etc. The above list is in no way intended to be limiting and exhaustive.

The exhaust apparatus 10 includes the blower that includes a motor 30 and fan 32 combination. The output of the blower vents through a series of openings which define an outlet 14 on the apparatus 10. The blower, in particular the motor fan assembly, is contained inside housing 16 positioned between the inlet 12 and the outlet 14, and is configured to draw air through the exhaust apparatus 10 from the inlet 12 to the outlet 14.

The air flow through the exhaust apparatus 10 of Figure 1 is shown illustratively via the dashed lines A in Figure 3.

The exhaust apparatus 10 includes at least one power source, which is typically at least one battery 34. The battery 34 can be any commercially-available battery 34, although the skilled person will appreciate that a compromise is always needed in terms of size and weight of the battery 34, and the capacity and duration of the battery 34. To control the operation of the blower, a switch mechanism 18 is accessible to the wearer 100. The switch mechanism can have a simple on/off mode of operation or can include a variable adjustment so that the wearer 100 can optimize the desired cooling effect based upon the environmental conditions, the task the wearer 100 is undertaking and personal choice.

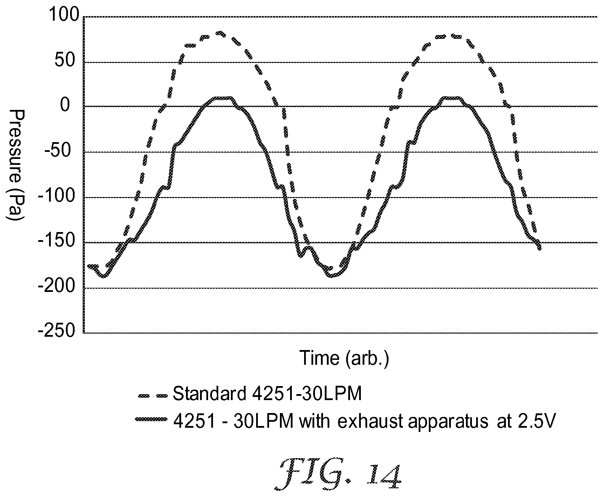

The operation of the exhaust apparatus 10 of Figure 1 is further, schematically illustrated in Figure 4 which shows a sectional side view of the exhaust apparatus 10 being operable to draw a portion of the wearer's 100 exhaled breath through an exhaust valve 26 on the personal protection respiratory device 20. The illustrative air flow through the respiratory mask 20 and exhaust apparatus 10 being denoted by arrows A. For sedentary tasks, a noticeable cooling effect is experienced by the wearer 100 when the blower is configured to operate at a volumetric flow rate of between 0 to 50 litres per minute through the exhaust valve 26. For arduous work, the blower may be configured to operate at a volumetric flow rate of over 180 litres per minute through the exhaust valve 26. The best perceived effect, in terms of battery life and cooling effect, occurs when the blower matches, or slightly exceeds, the peak exhalation flow rate of the wearer, as shown in Figure 14.

Further illustrations of the exhaust apparatus 10 of Figure 1 are shown in Figures 5 to 7. These show just how a purpose-designed apparatus 10 can be produced which is small, lightweight and balanced on the mask 20. Different designs of apparatus 10 are envisaged and different purpose-designed exhaust apparatuses 10 could also be styled to complement their respective negative pressure respirators 20, which all work in accordance with the mode of operation described herein.

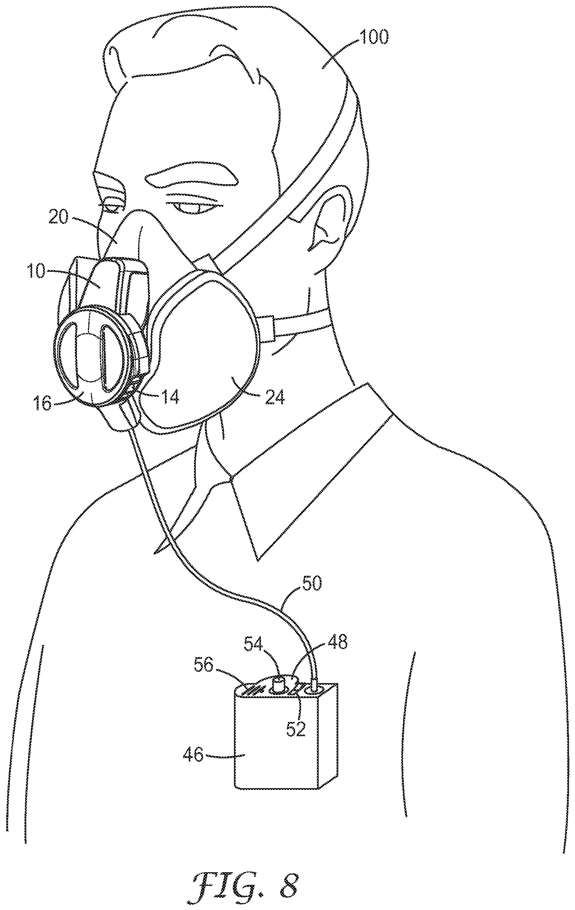

Figure 8 shows a front side perspective view of an exemplary exhaust apparatus 10 connected to a personal protection respiratory device 20, and further showing a remotely positionable battery pack 46. Figure 8 shows that the apparatus 10 can be configured with a breast pocket-mounted battery pack 46 that incorporates controls, such as an on/off switch 52 and speed adjuster 54, and display 56. By being breast pocket-mounted, and which attach to a wearer's clothing via clip 48, the controls are located in an easy to operate position and the visual display 56 showing battery life is located within the field of view of the wearer 100. The breast pocket-mounted battery pack 46 is connected to the blower in exhaust apparatus 10 via a wired connection 50.

On many respiratory masks 20, especially disposable respirators, it is obviously desirable to have a separate battery pack 46 to reduce the weight and or the size of the exhaust apparatus 10. By having a separate battery 46, larger capacity batteries can be used, leading to a longer operational time. A full range of display 56 options can then be located in the battery pack 46. These can include basic-colored LEDs, LED bargraphs or alphanumeric displays. More complex Graphical User Interface options, including visual and aural alarms/status indicators for flow range, mask pressure, battery, and remaining run time could also be used.

Whilst Figure 8 shows that the remote battery pack 46 is breast-mounted this is in no was intended to be limited as any number of remotely positionable battery configurations are envisaged, such as, for example, belt or waist mounted, helmet or headband mounted, arm or clip mounted.

Exhaust apparatuses of the present invention further include a secondary exhalation valve positioned between the inlet of the blower and the motor fan assembly.

Figure 9 shows a sectional side view of an exemplary exhaust apparatus 10 according to the present invention including a secondary exhalation valve 58 which reduces the exhalation pressure drop when the exhaust apparatus 10 is not powered or if exhaled air flow rate exceeds the amount of air flowing through the exhaust apparatus 10. The skilled person will appreciate that when the exhaust apparatus 10 is in operation it creates a cooling air flow inside a negative pressure respirator 20. However, when the unit 10 is attached to a respirator 20 and it is not powered, the extra resistance created by the flow of the exhaled air through both the exhaust valve 26 and the apparatus 10 can increase exhalation breathing resistance.

Respirators 20 such as those fitted with combined particulate and gas and vapor filters, can particularly exhibit a notable increase in the exhalation pressure drop when the exhaust apparatus 10 is not in operation. This is because the exhaled air has to pass through both the respirator exhalation valve 26 and the apparatus 10 and because the respirator 20 is fitted with inhalation valves to prevent exhaled air flowing back though the carbon filters 22, 24. The addition of a secondary exhalation valve 58, through exhaust vents 60, in the exhaust apparatus 10 serves to reduce the exhalation pressure drop when the apparatus 10 is not powered. By including a secondary exhalation valve 58 to the apparatus 10, positioned between the inlet 12 of the blower and the motor fan assembly 30, 32 this pressure drop can be reduced. Such a configuration means that the wearer 100 can benefit from the cooling air flow when the blower is activated, without the disadvantage of significantly increased exhalation pressure drop when the blower is switched off. The secondary exhalation valve 58 comprises a valve seat that includes a seal surface and a flexible flap, although other configurations are, of course, possible.

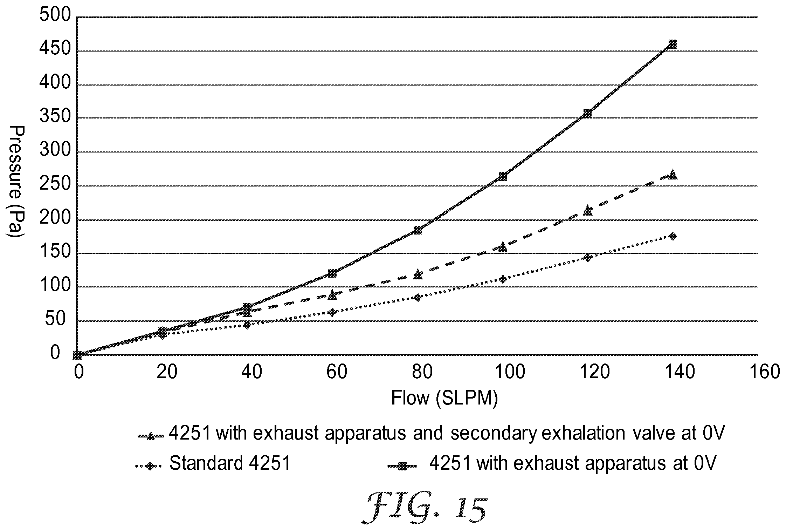

Figure 9 shows the exhalation flow path for an exhaust apparatus fitted with an extra exhalation valve 58. In this diagram, you can see that when the blower is switched off, the air passes though the secondary exhalation valve 58 and not the blower of the exhaust apparatus 10. This secondary exhalation valve 58 significantly reduces the exhalation pressure drop, as described below in relation to Figure 15.

The change in exhalation pressure drop has been determined by conducting constant flow tests through a standard 3M^{™} 4251 Valved Filtering Half Face Respirator, a 3M^{™} 4251 Valved Filtering Half Face Respirator fitted with an exhaust apparatus 10, and a 3M^{™} 4251 Valved Filtering Half Face Respirator fitted with an exhaust apparatus 10 including an additional exhalation valve 58. The exhalation pressure drop for all three configurations was measured by conducting constant flow tests with the respirators fitted to a Sheffield test headform. All the measurements taken in Figure 15 were obtained with the blower of the exhaust apparatus 10 not powered. With the power turned off to the exhaust apparatuses 10, the exhalation pressure drop is significantly improved for an apparatus 10 that includes a secondary exhalation valve 58, as the exhaled air passes though the secondary exhalation valve 58 and not through the blower and outlet 14 of the exhaust apparatus 10, as shown schematically in Figure 9.

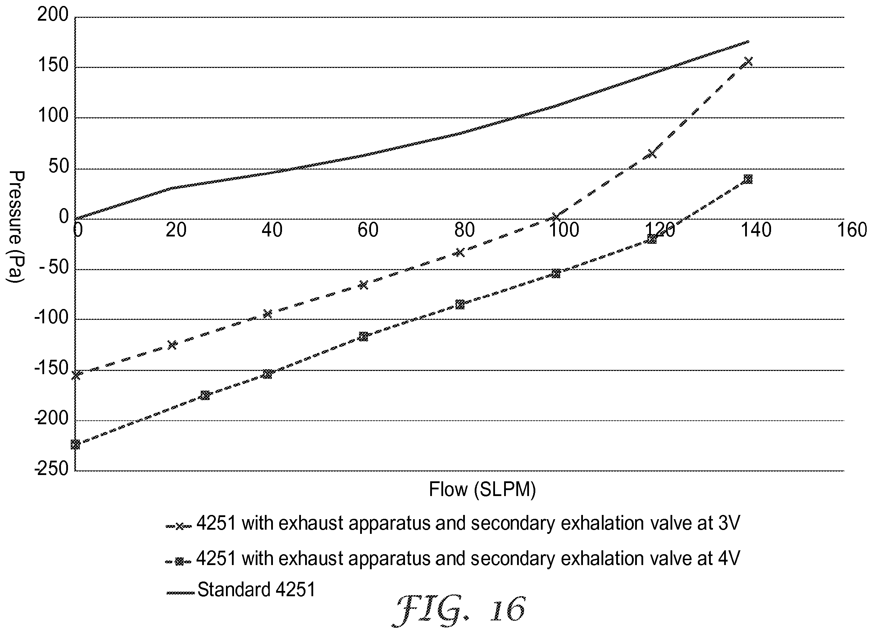

Figure 16 illustrates the measured exhalation pressure drop using a 3M^{™} 4251 Valved Filtering Half Face Respirator having an exhaust apparatus 10 connected thereto as a function of flow rate and applied voltage. The solid line in Figure 16 is the measured exhalation pressure drop for a standard 3M^{™} 4251 Valved Filtering Half Face Respirator measured against flow rate. Figure 16 shows that there is a significant drop in exhalation pressure drop as the voltage to the blower is increased. This is to be expected since the exhaust apparatus 10 draws air out through the blower and reduces exhalation resistance. In use, for the wearer 100 this means it is easier to exhale and it is the continual assisted removal of the hot and moist air inside the respirator 20 through the blower that produces a noticeable cooling effect.

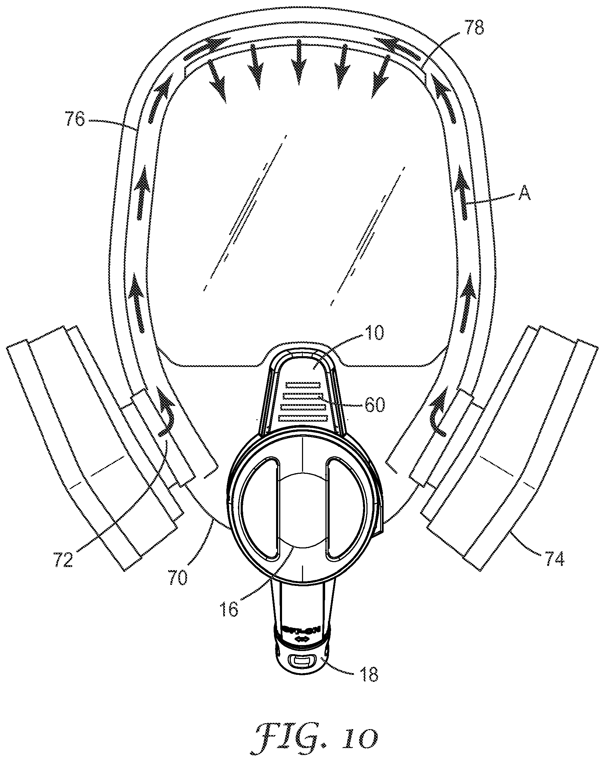

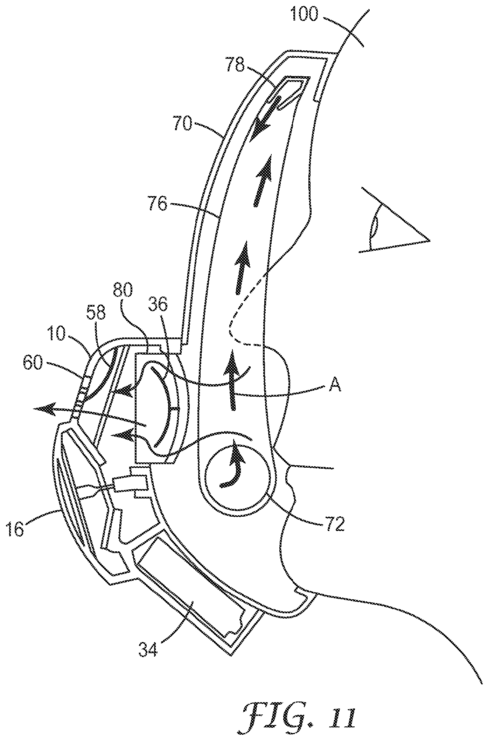

Figures 10 and 11 illustrate how an exhaust apparatus 10 according to the present invention can be utilized with a full facepiece respiratory device 70. The respirator 70 that is illustrated in Figures 10 and 11 is indicative of the 3M^{™} 6800 Full Facepiece Reusable Respirator manufactured by 3M Company located in St. Paul, Minnesota. As shown in Figures 10 and 11, filter cartridges 74 are attached at either side of the respirator mask 70 at respective inhalation ports 72. Each of the inhalation ports 72 has a respective inhalation valve (not shown) located on the inside of the respirator mask 70 which open as a wearer 100 draws a breath. The face mask 70 includes an exhaust valve 80 with a one-way exhalation valve diaphragm 36, and adjustable straps (not shown) for attachment to the wearer 100.

The respiratory mask 70 has a conformable gasket or seal which generally encloses the wearer's 100 face. Since a good seal is needed to ensure filtration of the containments one major drawback is that sometimes an uncomfortable build-up of heat and moisture is noticed by the wearer 100 inside the respirator 70. As the wearer 100 works harder, and or wears the respirator 70 for extended periods of time, heat and moisture build-up can occur. The heat and moisture build-up is caused by the trapping of the exhaled breath in the cavity created between the respirator 20 and the wearer's 100 face. In a full facepiece respirator 70 the build-up of trapped hot and moist air can also cause the additional problem of visor misting.

As described above, the exhaust apparatus 10 of the present invention is operable to draw a portion of the wearer's 100 exhaled breath through the one-way exhalation valve diaphragm 36 on the personal protection respiratory device 70 to significantly improve and enhance wearer comfort. Figures 10 and 11 also show how a standard full facepiece respiratory device 70 can be modified to more effectively control or direct the air flow inside the respirator 70 to give even better improvements in terms of visor misting and the cooling effect experienced by the wearer 100.

The respiratory device 70 shown in Figures 10 and 11 also includes an additional air distribution manifold 76 that is connected to each of the inhalation ports 72. Located generally above the wearer's 100 eye line is the manifold outlet 78. The air flow through the respiratory device 70 and exhaust apparatus 10 is shown illustratively via the bold lines A in Figures 10 and 11. As can be seen, as the wearer 100 draws a breath, negative pressure is created in the respirator 70 and air is drawn in through the filter system, comprising the inhalation ports 72, filter cartridges 74, air distribution manifold 76, and the air exits at inside the mask 70 at the manifold outlet 78. The air is then drawn downwards towards the nose and mouth of the wearer 100. When the wearer 100 exhales a breath, spent air is drawn out of the one-way exhalation valve diaphragm 36 in the respirator 70 by the exhaust apparatus 10. By having such a directional air flow inside the mask 70, with the "cooler" ambient air being drawn towards the top of the respiratory mask 70 and then downwards across both the visor of the respiratory mask 70 and the wearer's 100 face, this gives an enhanced cooling effect for the wearer 100 and further improvements in terms of preventing visor misting.

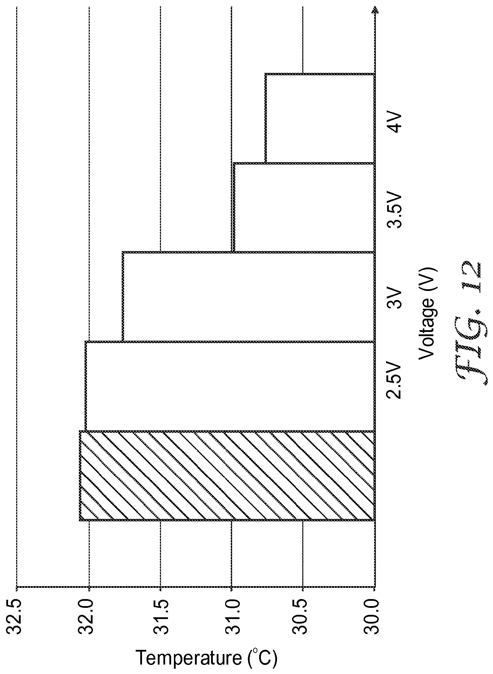

The cooling effect achieved from the exhaust apparatus 10 is further illustrated in Figure 12, which shows the average temperature measured inside a 3M^{™} 4251 Valved Filtering Half Face Respirator as a function of the voltage being applied to the exhaust apparatus 10. The results shown in Figure 12 were again obtained using standard respiratory protection test equipment and the respirator 20 was fitted to a Sheffield test head and breathing machine capable of providing a number of pre-set swept volumes of air at variable rates up to 50 strokes per minute. The output of the breathing machine was connected to an enclosed box containing a volume of water and a heater element such that the air is warmed and moistened before connection to the Sheffield test headform, which carried the respirator 20 under test. A thermocouple was placed inside the respirator, in the air volume adjacent to the wearer's 100 nose and mouth and Figure 12 shows the average temperature inside 3M^{™} 4251 Valved Filtering Half Face Respirator. The temperate readings were each averaged over 5 minute intervals and shows a continuous test run.

As can be seen, the average temperature inside the standard respirator is around 32.1°C as the test commences. This is illustrated by the shaded block at the left hand side of Figure 12. As described above, this is because the exhaled air has to pass through both the respirator exhalation valve 26 and the apparatus 10. The 3M^{™} 4251 Valved Filtering Half Face Respirator, which is fitted with combined particulate and gas and vapor filters, can particularly exhibit a notable increase in the exhalation pressure drop when the apparatus 10 is not in operation. It is only when the supplied voltage to the exhaust apparatus is increased that a corresponding decrease in the temperature inside the mask is observed. To conclude the test, the exhaust apparatus was then removed and a measurement of the standard 3M^{™} 4251 Valved Filtering Half Face Respirator was taken to confirm that the temperature of the supplied air had remained constant during the test.

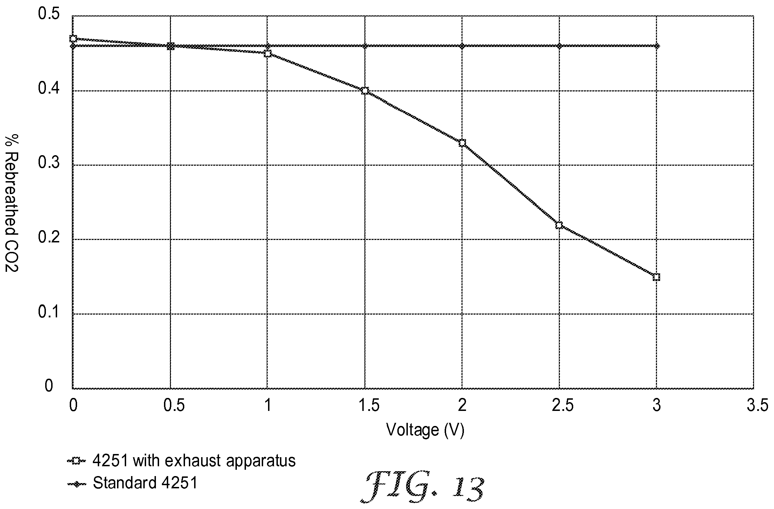

As well as reducing the temperature inside the respirator 20, the use of an exhaust apparatus 10 also gives a significant reduction in the rebreathed carbon dioxide levels observed inside the respirator, as shown in Figure 13. These measurements were again obtained using standard respiratory protection test equipment with the 3M^{™} 4251 Valved Filtering Half Face Respirator being fitted to a Sheffield test head using a breathing machine, and an apparatus to provide warm moist exhaled air. These tests being in accordance with EN 405:2001, paragraphs 7.14 and 8.8. Figure 13 shows that as well as observing a significant reduction in the temperature observed inside the respirator 20, the measured carbon dioxide levels in front of the wearer's 100 mouth and nose are reduced as the voltage to the exhaust apparatus 10 increases.

This is because the apparatus 10 draws out the last portion of the wearer's previously exhaled breath so that the first portion of the next breath of the wearer 100 is fresh un-breathed air. Apart from there being stringent regulations on the absolute levels of carbon dioxide concentration, which the standard 3M^{™} 4251 Valved Filtering Half Face Respirator clearly meets, this reduction in rebreathed carbon dioxide levels observed by using the exhaust apparatus 10 will also enhance wearer comfort.

The principle of operation, and the cooling effect achieved by the exhaust apparatus 10 can be further understood from Figure 14. Figure 14 is a graph of the measured pressure inside a standard 3M^{™} 4251 Valved Filtering Half Face Respirator using a breathing machine set at 30 litres per minute, compared to a 3M^{™} 4251 Valved Filtering Half Face Respirator having an exhaust apparatus 10 connected thereto. Again these measurements were taken using standard respiratory protection test equipment with the 3M^{™} 4251 Valved Filtering Half Face Respirator being fitted to a Sheffield test head and breathing machine.

Figure 14 shows the measured pressure inside the respirator 20 as the pneumatic cylinder of the breathing machine provides a pre-set swept volume of air in and out of the respirator 20 and is a simulation of a breathing cycle. For the standard 3M^{™} 4251 Valved Filtering Half Face Respirator when the pressure is above 0 Pa this is indicative of the exhale phase of breathing, when hot and moist air is being introduced by the wearer into the mask 20. When the line is below 0 Pa, this is indicative of the inhalation phase of the breathing cycle, when "cooler" ambient air is drawn into the respiratory mask 20 either though the filter cartridges 24, 26 as shown in Figures 1 and 2 for a reusable mask, or through, for example, a filter portion or filtering mask body of the respirator 20, as with a disposable mask. The addition of the exhaust apparatus 10 being run at 2.5 V clearly shifts the breathing cycle towards the "cooler" parts of the breathing cycle below 0 Pa. The pressure on exhalation has been reduced at 2.5 V without adding an increase to the inhalation pressure drop. The optimum results are obtained when the exhaust apparatus pressure removes all of the exhaled air inside the mask. This occurs when the peak pressure inside the mask is zero, or below zero, at the peak exhalation flow rate of the wearer, as is shown in Figure 14.

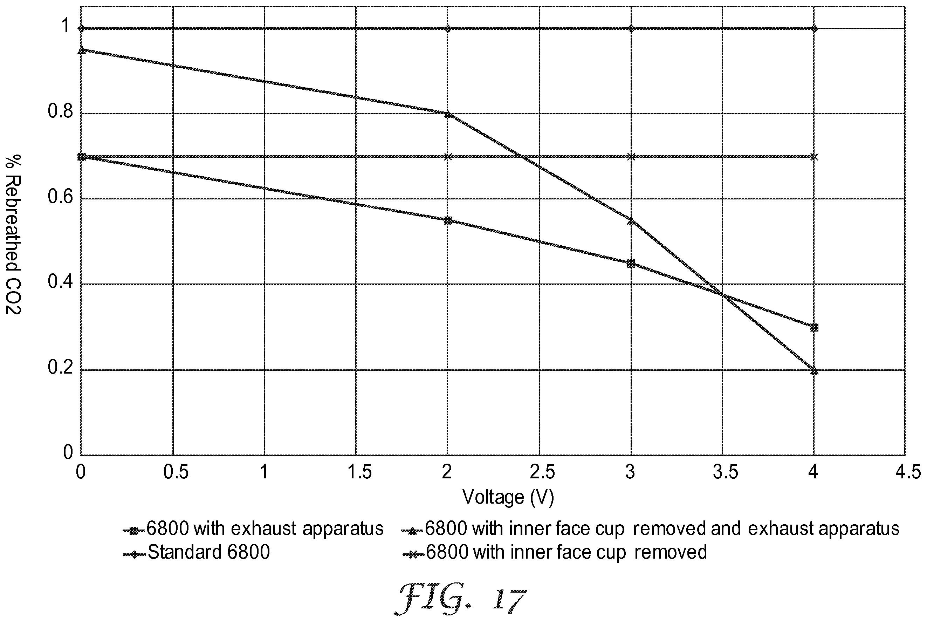

Figure 17 illustrates that the use of an exhaust apparatus 10 gives a significant reduction in the rebreathed carbon dioxide levels observed inside a full facepiece respiratory device 70. These measurements were obtained using standard respiratory protection test equipment with a 3M^{™} 6800 Full Facepiece Reusable Respirator being fitted to a Sheffield test head using a breathing machine. These tests being in accordance with EN 136:1998, paragraphs 7.18 and 8.14. Figure 17 shows that as well as observing a significant reduction in the temperature observed inside the respirator 20, the measured carbon dioxide levels in front of the wearer's 100 mouth and nose are reduced as the voltage to the exhaust apparatus 10 increases.

This is because the apparatus 10 draws out the last portion of the wearer's previously exhaled breath so that the first portion of the next breath of the wearer 100 is fresh un-breathed filtered air. Figure 17 also shows that if the inner face cup is removed from the respirator 70 leaving a totally open space encompassing the wearer's 100 face sealed only by the outer conformable gasket or seal then improvements in terms of rebreathed carbon dioxide levels are also observed as the voltage applied to the exhaust apparatus 10 are increased. By directing the air flow inside the respirator 70 by virtue of the air distribution manifold 76 and manifold outlet 78, as described above in relation to Figures 10 and 11, this can give even better improvements in terms of preventing visor misting and the cooling effect experienced by the wearer whilst exceeding the relevant regulatory requirements for the carbon dioxide content of the inhaled air without an inner face cup, which also increases the wearer's 100 field of view.

Various alterations and modifications may be made to the present invention without departing from the scope of the claims. For example, although particular examples refer to implementing the present invention with respirators fitted with combined particulate and gas and vapor filters, this is in no way intended to be limiting as, in use, the present invention has been implemented and utilized with any negative pressure respiratory mask including, but not limited to disposable, reusable, half mask, full face, gas and vapour and tight-fitting hood respirators.

## Claims

1. An exhaust apparatus (10) for releasable connection to a personal protection respiratory device (20) that defines a filtered air volume adjacent to the face of a wearer (100) and comprises at least one exhalation valve (26), the exhaust apparatus (10) comprising:
a blower comprising an inlet (12), a motor fan assembly (30, 32), and an outlet (14);
an attachment means for releasably connecting the blower to the at least one exhalation valve (26), the blower being in fluid connection with the at least one exhalation valve (26) when releasably connected thereto and being operable to draw a portion of the wearer's exhaled breath through the at least one exhalation valve (26); **characterized by**
a secondary exhalation valve (58) positioned between the inlet (12) of the blower and the motor fan assembly (30, 32).

2. The exhaust apparatus (10) as claimed in claim 1, wherein the attachment means is selected from a group consisting of interference fit, screw thread, snap fit engagement, bayonet, quick release mechanism, slider and groove engagement, locking pin, locking clip and mechanical hook and loop fastener.

3. The exhaust apparatus (10) as claimed in claim 1, wherein the personal protection respiratory device (20) is selected from a group consisting of disposable, reusable, half mask, full face, particulate, gas and vapour and tight-fitting hood respirators.

4. The exhaust apparatus (10) as claimed in claim 1, wherein the blower is operable at a volumetric flow rate of between 0 to 180 litres per minute.

5. The exhaust apparatus (10) as claimed in claim 1, wherein the blower is in fluidic connection with at least one exhalation valve (26) via a breathing hose, tube, pipe, duct or channel.

6. The exhaust apparatus (10) as claimed in claim 1, wherein the secondary exhalation valve is integrally formed with the exhaust apparatus.

7. The exhaust apparatus (10) as claimed in claim 1, wherein the secondary exhalation valve comprises a valve seat that includes a seal surface and a flexible flap.

## Patentansprüche

1. Eine Abführungseinrichtung (10) für eine lösbare Verbindung mit einem persönlichen Atemschutzgerät (20), das ein gefiltertes Luftvolumen angrenzend an dem Gesicht eines Trägers (100) definiert und mindestens ein Ausatmungsventil (26) aufweist, die Abführungseinrichtung (10) aufweisend:
ein Gebläse, aufweisend einen Einlass (12), eine Motorlüfteranordnung (30, 32) und einen Auslass (14);
ein Befestigungsmittel zum lösbaren Verbinden des Gebläses mit dem mindestens einen Ausatmungsventil (26), wobei das Gebläse in Fluidverbindung mit dem mindestens einen Ausatmungsventil (26) steht, wenn es lösbar damit verbunden ist, und betreibbar ist, um einen Abschnitt der ausgeatmeten Luft des Trägers durch das mindestens eine Ausatmungsventil (26) zu ziehen;
**gekennzeichnet durch**
ein sekundäres Ausatmungsventil (58), das zwischen dem Einlass (12) des Gebläses und der Motorlüfteranordnung (30, 32) positioniert ist.

2. Die Abführungseinrichtung (10) nach Anspruch 1, wobei das Befestigungsmittel aus einer Gruppe ausgewählt ist, bestehend aus Presspassung, Schraubgewinde, Schnappeingriff, Bajonett, Schnelllösemechanismus, Schieber- und Nuteingriff, Verriegelungsstift, Verriegelungsclip und mechanischem Klettverschluss.

3. Die Abführungseinrichtung (10) nach Anspruch 1, wobei das persönliche Atemschutzgerät (20) aus einer Gruppe ausgewählt ist, bestehend aus Einweg-, wiederverwendbaren, Halbmasken-, Vollgesichts-, Partikel-, Gas- und Dampf- und eng anliegenden haubenartigen Atemgeräten.

4. Die Abführungseinrichtung (10) nach Anspruch 1, wobei das Gebläse mit einer Volumenstromrate von zwischen 0 bis 180 Liter pro Minute betreibbar ist.

5. Die Abführungseinrichtung (10) nach Anspruch 1, wobei das Gebläse über einen Beatmungsschlauch, ein Beatmungsrohr, eine Beatmungsleitung, eine Beatmungsleitungsröhre oder einen Beatmungskanal mit mindestens einem Ausatmungsventil (26) in Fluidverbindung steht.

6. Die Abführungseinrichtung (10) nach Anspruch 1, wobei das sekundäre Ausatmungsventil mit der die Abführungsvorrichtung einstückig ausgebildet ist.

7. Die Abführungseinrichtung (10) nach Anspruch 1, wobei das sekundäre Ausatmungsventil einen Ventilsitz umfasst, der eine Dichtungsoberfläche und eine flexible Klappe einschließt.

## Revendications

1. Appareil d'échappement (10) pour une liaison amovible à un dispositif respiratoire de protection individuelle (20) qui définit un volume d'air filtré adjacent au visage d'un porteur (100) et comprend au moins une soupape d'expiration (26), l'appareil d'échappement (10) comprenant :
une soufflante comprenant une entrée (12), un ensemble moteur-ventilateur (30, 32) et une sortie (14) ;
un moyen de fixation pour relier de manière amovible la soufflante à l'au moins une soupape d'expiration (26), la soufflante étant en liaison fluidique avec l'au moins une soupape d'expiration (26) lorsqu'elle y est reliée de manière amovible et pouvant être utilisée pour aspirer une partie de l'air expiré par le porteur à travers l'au moins une soupape d'expiration (26) ; **caractérisé par**
une soupape d'expiration secondaire (58) placée entre l'entrée (12) de la soufflante et l'ensemble moteur-ventilateur (30, 32).

2. Appareil d'échappement (10) selon la revendication 1, dans lequel le moyen de fixation est choisi parmi un groupe constitué d'un ajustement serré, d'un filetage de vis, d'une mise en prise par ajustement encliquetable, d'une baïonnette, d'un mécanisme à libération rapide, d'une mise en prise à glissière et rainure, d'une goupille de verrouillage, d'une attache de verrouillage et d'un fermoir mécanique à crochets et boucles.

3. Appareil d'échappement (10) selon la revendication 1, dans lequel le dispositif respiratoire de protection individuelle (20) est choisi parmi un groupe constitué de respirateurs jetables, réutilisables, à demi-masque, pour visage complet, particulaires, pour gaz et vapeur et de type cagoule à ajustement serré.

4. Appareil d'échappement (10) selon la revendication 1, dans lequel la soufflante est opérationnelle à un débit volumique compris entre 0 et 180 litres par minute.

5. Appareil d'échappement (10) selon la revendication 1, dans lequel la soufflante est en liaison fluidique avec au moins une soupape d'expiration (26) par l'intermédiaire d'un tuyau, d'un tube, d'une conduite, d'un conduit ou d'un canal respiratoire.

6. Appareil d'échappement (10) selon la revendication 1, dans lequel la soupape d'expiration secondaire est formée d'un seul tenant avec l'appareil d'échappement.

7. Appareil d'échappement (10) selon la revendication 1, dans lequel la soupape d'expiration secondaire comprend un siège de soupape qui comporte une surface d'étanchéité et un clapet flexible.
